# EUROPEAN PATENT APPLICATION

(11) **EP 2 168 941 A1**
(43) Date of publication of application: **31.03.2010**
(21) Application number: 08305618.4
(22) Date of filing: 30.09.2008
(51) Int. Cl.: C07C 69/708, C11B 9/00

(54) **Novel Glycolate derivatives**

(71) Applicant: V. Mane Fils, 06620 Bar sur Loup (FR)
(72) Inventor: Plessis, Caroline, 06740 Chateauneuf (FR); Mane, Jean, 06130 Grasse (FR); Chanot, Jean-Jacques, 06530 Speracedes (FR)
(74) Representative: Cabinet Plasseraud

(57) **Abstract**

The invention relates to compounds of formula (I) 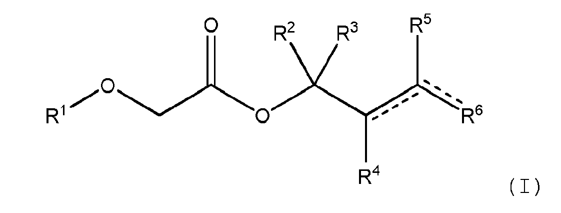 and their use in the field of perfumery.

## Description

### [FIELD OF THE INVENTION]

The present invention relates to the field of fragrances and flavours. More particularly, the invention relates to new glycolate derivatives and their use in the fields of perfumery and flavouring.

### [BACKGROUND]

Allyl glycolates belong to an important class of fragrant ingredients and much work has already been done to prepare known compounds, such as Cyclogalbanate® and allyl phenoxy acetate and similar derivatives. These compounds have a characteristic green (galbanum) and/or fruity (pineapple) note. Bajgrowicz et al. describe a series of green/galbanum type smelling derivatives with a fruity undertone, including the known allyl glycolates (Bajgrowicz et al., Bioorg. Med. Chem. 11 (2003) 2931-2946).

### [PROBLEM TO BE SOLVED]

The need for new compounds is of great importance for the development of the fragrance industry, which recently had to face stricter international regulatory requirements about the use of certain materials, as well as environmental concerns and customer demands for improved performance. Developing new fragrant and/or flavouring compounds is also important for providing alternatives to already existing fragrant and/or flavouring compounds so as to minimize the risk of allergies due to repeated exposure to the same compounds. Providing new fragrant and/or flavouring compounds as well as means of manufacturing such compounds, is therefore an object of the invention.

In other words, it is an aim of the present invention to provide new fragrant compounds having a green note, in particular unsaturated glycolates.

### [SUMMARY OF THE INVENTION]

The invention is directed to novel compounds of formula (I) 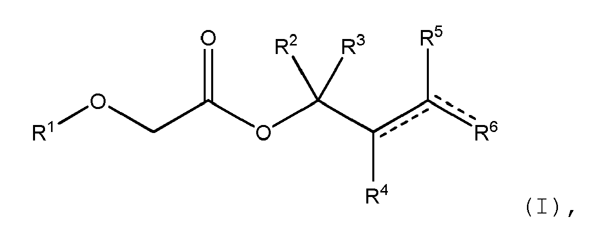 wherein
R¹ is C₆-C₁₂ alkenyl or a saturated or unsaturated C₆ carbocyclic group, wherein the carbocyclic group is substituted with at least one alkyl or alkenyl group,
R², R³, R⁴, R⁵, and R⁶ are independently H, C₁-C₅ alkyl, or C₂-C₅ alkenyl;
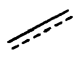 is a single or double bond, provided that one of the 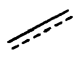 is a single bond and the other one is a double bond;
provided that if R⁶ is attached to the double bond, it is H, C₁-C₅ alkyl, or C₂-C₅ alkenyl; and
if R⁶ is part of the double bond, it is =CR'R", wherein R' and R" are independently H, C₁-C₄ alkyl, or C₂-C₄ alkenyl, with the proviso that R' and R" together form at most a C₄-alkyl or C₄-alkenyl group.

The compounds of the invention exhibit interesting olfactive properties, such as fresh green notes with sometimes animalic, leathery or musky aspects. The compounds of the invention are therefore of particular interest in the field of perfumery.

### [DETAILED DESCRIPTION OF THE INVENTION]

As noted above, the invention relates to compounds of formula (I) 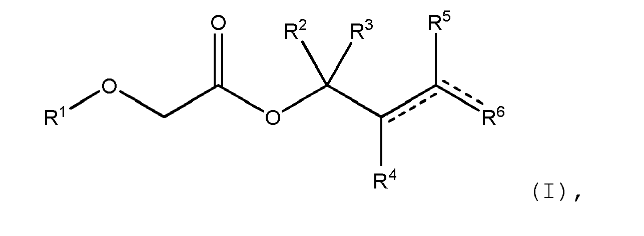 wherein
R¹ is C₆-C₁₂ alkenyl or a saturated or unsaturated C₆ carbocyclic group, wherein the carbocyclic group is substituted with at least one alkyl or alkenyl group,
R², R³, R⁴, R⁵, and R⁶ are independently H, C₁-C₅ alkyl, or C₂-C₅ alkenyl;
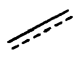 is a single or double bond, provided that one of the 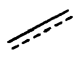 is a single bond and the other one is a double bond;
provided that if R⁶ is attached to the double bond, it is H, C₁-C₅ alkyl, or C₂-C₅ alkenyl; and
if R⁶ is part of the double bond, it is =CR'R", wherein R' and R" are independently H, C₁-C₄ alkyl, or C₂-C₄ alkenyl, with the proviso that R' and R" together form at most a C₄-alkyl or C₄-alkenyl group.

The invention includes all isomers of the compounds of formula (I).

In one embodiment, R¹ is a cyclohexyl or cyclohexenyl group, preferably a cyclohexyl group, substituted with at least one alkyl or alkenyl group, preferably with 1 to 4 (i.e. 1, 2, 3, or 4) groups independently selected from C₁-C₆ alkyl and C₂-C₆ alkenyl.

Advantageously, R¹ is selected from the group consisting of 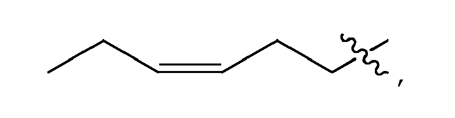 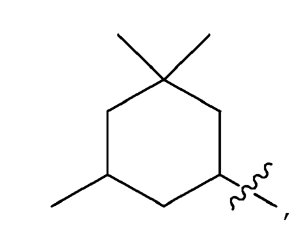 and

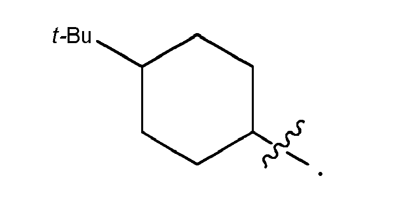

In one advantageous embodiment R², R³, R⁴, R⁵, and R⁶ are H. In another advantageous embodiment R², R³, R⁴, and R⁵ are H and R⁶ is =CR'R", wherein R' is H and R" is =CH-CH₂-CH₃.

Particularly interesting compounds of formula (I) are (*3Z*)-Hex-3-enyloxy-acetic acid allyl ester, (4-tert-Butyl-cyclohexyloxy)-acetic acid allyl ester, (3,3,5-Trimethyl-cyclohexyloxy)-acetic acid allyl ester, and (*3Z*)-Hex-3-enyloxy-acetic acid (*3*Z*)*-3-hexenyl ester.

The compounds of the invention exhibit interesting olfactive properties. Very unexpectedly and surprisingly, they do not show the typical Galbanum type green note that the skilled person would have expected, but rather a fresh green note with sometimes other undertones, such as animal, leathery or even musky notes.

A further object of the invention is thus the use of a compound of formula (I) as defined above, for the preparation of perfumed bases and concentrates, fragrances, perfumes; topic compositions; cosmetic compositions, such as face and body creams, cleansers, facial treatments, talc powders, hair oils, shampoos, hair lotions, bath oils and salts, shower and bath gels, soaps, body anti-perspirants and deodorizers, pre-shave, shaving and post-shave creams and lotions, creams, toothpastes, mouth baths, pomades; and cleaning products, such as softeners, detergents, air deodorizers and household cleaning supplies.

The invention is also directed to the use of a compound of formula (I) as defined above, as flavouring agent for the preparation of foodstuffs, drinks, and tobacco. The foodstuffs and drinks are preferably selected from the group consisting of dairy products, ice creams, soups, sauces, dips, dishes, meat products, culinary assistances, salted biscuits, snacks, soft drinks, beers, wines and spirits.

The invention is also directed to the use of a compound of formula (I) as defined above, as masking agent of odours and/or flavours, e.g. in pharmaceutical, cosmetic or food compositions.

The invention also provides the use of a compound of formula (I) as defined above, in combination with other perfuming or flavouring ingredients, solvents or additives or fixatives.

The compounds of the invention may be used in a concentration comprised in a range from 0.001% to 99% in weight, preferably from 0.1% to 50% in weight, more preferably from 0.1% to 30% in weight. It is known by the man skilled in the art that these values depend on the nature of the composition/article to be perfumed and/or flavoured, the desired intensity of the perfume and/or flavour, and of the nature of the other ingredients present in said composition or article. According to a preferred embodiment of the invention, compounds are used in an olfactory effective amount.

### [DEFINITIONS]

The terms "fragrance" and "fragrant", as used herein, are used interchangeably whenever a compound or a mixture of compounds is referred to, which is intended to pleasantly stimulate the sense of smell.

The terms "flavour" and "flavouring", as used herein, are used interchangeably whenever a compound or a mixture of compounds is referred to, which is intended to stimulate the sense of taste and smell. Also in the meaning of the invention, the term "flavouring" relates to the flavouring of any liquid or solid, human or animal, in particular of drinks, dairy products, ice creams, soups, sauces, dips, dishes, meat products, culinary assistances, salted biscuits or snacks. It also means the flavouring of beers, wines and tobaccos.

The term "olfactory effective amount", as used herein, means a level or amount of fragrant/flavouring compound present in a material at which the incorporated compound exhibits a sensory effect.

By the term "masking" is meant reducing or eliminating malodour or bad flavour perception generated by one or more molecules entering in the composition of a product.

The term "isomer", in the present invention, means molecules having the same chemical formula, which means same number and types of atoms, but in which the atoms are arranged differently. The term "isomer" includes structural isomers, geometric isomers, optical isomers and stereoisomers. It particularly includes the Z/E isomers of the compounds of formulae (I), (Ia), and (Ib).

The term "alkyl" or "alkyl group", in the present invention, means any linear or branched saturated hydrocarbon chain, having preferably 1, 2, 3, 4 or 5 carbon atoms and herein referred to as C₁₋₅ alkyl group, such as for example methyl, ethyl, n-propyl, isopropyl, n-butyl, sec-butyl, *iso*-butyl, tert-butyl, and pentyl.

The term "alkenyl" or "alkenyl group", in the present invention, means any linear or branched mono or poly unsaturated hydrocarbon chain, having preferably 2, 3, 4 or 5 carbon atoms and herein referred to as C₂₋₅ alkenyl group, such as for example ethenyl, prop-1-enyl, allyl, but-1-enyl, but-2-enyl, or pentenyl.

The present invention will be better understood with reference to the following examples. These examples are intended to representative of specific embodiments of the invention, and are not intended as limiting the scope of the invention.

### Example 1: (3Z)-Hex-3-enyloxy-acetic acid allyl ester

Step 1: cis-3-Hexenol is added dropwise to a suspension of sodium hydride in THF and the mixture is further stirred under reflux for 3 hours. After cooling down to room temperature, chloroacetic acid sodium salt is added portionwise and the reaction mixture is further reacted under reflux overnight.

It is then poured into a 50:50 water/t-butyl methyl ether mixture and the aqueous phase is acidified and extracted three times with t-butyl methyl ether. The extracted layers are washed with water and with brine and dried over magnesium sulphate. The solvents are evaporated to give crude hex-3-enyloxy-acetic acid.

Step 2: A solution of hex-3-enyloxy-acetic acid in cyclohexane with a catalytic amount of PTSA is heated at 80°C and allylic alcohol is added dropwise. After removal of the formed water using a Dean-Stark apparatus, the solution is cooled down, washed with a saturated aqueous sodium bicarbonate solution and with brine, and dried over magnesium sulphate. The solvents are evaporated and the crude product is purified by distillation to give (*3Z*)-hex-3-enyloxy-acetic acid allyl ester.

Using the E isomer of alcohol or a Z/E mixture of alcohol leads to the respective E or E/Z allyl ester. Odour description: Green, cut grass, watery, clean (Ironed linen), powerful.

### Z-Isomer:

¹H-NMR (200 MHz, CDCl₃) δ (ppm) 0.95 (t, *J* = 7.5 Hz, 3H), 1.94-2.13 (m, 2H), 2.24-2.46 (m, 2H), 3.53 (t, *J* = 7.1 Hz, 1H), 4.06 (s, 2H), 4.08-4.12 (m, 2H), 4.14 (t, *J* = 7.0 Hz, 1H), 5.15-5.37 (m, 3H), 5.37-5.60 (m, 1H), 5.79-6.02 (m, 1H).

¹³C-NMR (50 MHz, CDCl₃) : δ (ppm) 14.13, 20.53, 26.63, 64.23, 67.04, 72.29, 118.10, 123.29, 133.69, 134.73, 170.34.

IR (film, cm⁻¹) : 723w, 930w, 993w, 1136s, 1197s, 1274m, 1429w, 1460w, 1738m, 1758s, 2876m, 2936m, 2965m, 3012w.

MS [e/m (%)]: 198 (M+, <1), 82 (86), 71 (20), 67 (83), 55 (57), 41 (100), 39 (30).

### E-Isomer:

¹H-NMR (200 MHz, CDCl₃) δ (ppm) 0.95 (t, *J* = 7.5 Hz, 3H), 1.94-2.13 (m, 2H), 2.24-2.46 (m, 2H), 3.62 (t, *J* = 6.5 Hz, 1H), 4.06 (s, 2H), 4.08-4.12 (m, 1H), 4.64 (td, *J* = 1.1 Hz, *J* = 5.7 Hz, 2H), 5.15-5.37 (m, 3H), 5.37-5.60 (m, 1H), 5.79-6.02 (m, 1H).

¹³C-NMR (50 MHz, CDCl₃) : δ (ppm) 14.16, 20.57, 27.60, 65.32, 68.21, 71.42, 118.71, 124.23, 131.66, 133.95, 170.10.

### Example 2: (4-tert-Butyl-cyclohexyloxy)-acetic acid allyl ester

It is prepared from 4-tert-butyl-cyclohexanol, chloroacetic acid sodium salt and allylic alcohol according to example 1.

It consists in a 10:90 *cis*/*trans* mixture of isomers.
Odour description: Leathery, burnt, green.

IR (film, cm⁻¹) : 929w, 987w, 1133s, 1190s, 1276w, 1366w, 1451w, 1737m, 1760s, 2865m, 2946s.

### trans-Isomer:

¹H-NMR (200 MHz, CDCl₃) : δ (ppm) 0.82 (s, 9H), 0.88-1.10 (m, 3H), 1.10-1.40 (m, 2H), 1.70-1.87 (m, 2H), 2.10-2.16 (m, 2H), 3.24 (tt, *J* = 4.3 Hz, *J* = 10.9 Hz, 1H), 4.13 (s, 2H), 4.63 (td, *J* = 1.3 Hz, *J* = 5.8 Hz, 2H), 5.19-5.37 (m, 2H), 5.91 (tdd, *J* = 5.8 Hz, *J* = 10.3 Hz, *J* = 17.1 Hz, 1H).

¹³C-NMR (50 MHz, CDCl₃) : δ (ppm) 25.47 (2C), 27.55 (3C), 32.23 (2C + 1C), 47.23, 65.29, 65.49, 79.44, 118.63, 131.76, 170.65.

MS [e/m (%) ] : 254 (M+, <1), 155 (49), 83 (31), 81 (36), 67 (26), 57 (100), 55 (24), 41 (63).

### cis-Isomer:

¹H-NMR (200 MHz, CDCl₃, selected data): δ (ppm) 3.57-3.65 (m, 1H), 4.08 (s, 2H).

¹³C-NMR (50 MHz, CDCl₃, selected data): δ (ppm) 21.29 (2C), 27.45 (3C), 30.27 (2C), 47.88, 65.20, 65.55, 74.26, 118.51, 131.80.

MS [e/m (%)] : 254 (M+, <1), 155 (48), 117 (21), 83 (29), 81 (44), 67 (44), 57 (100), 55 (28), 41 (71).

### Example 3: (3,3,5-Trimethyl-cyclohexyloxy)-acetic acid allyl ester

It is prepared from 3,3,5-trimethyl-cyclohexanol (Obtained from the reduction of Isophorone), chloroacetic acid sodium salt and allylic alcohol according to example 1.

It consists in a 93:7 *cis*/*trans* mixture of isomers.
Odour description: Costus, musky.

IR (film, cm⁻¹) : 931m, 987m, 1129s, 1183s, 1193s, 1275m, 1365w, 1388w, 1430w, 1457m, 1738m, 1761s, 2838w, 2870m, 2914m, 2951s, 2992w.

### cis-Isomer:

¹H-NMR (200 MHz, CDCl3) : δ (ppm) 0.71-1.14 (m, 3H), 0.85 (d, *J* = 6.4 Hz, 3H), 0.85 (s, 3H), 1.39 (ddd, *J* = 2.2 Hz, *J* = 5.2 Hz, *J* = 12.9 Hz, 1H), 1.72 (qd, *J* = 2.3 Hz, *J* = 14.4 Hz, 1H), 1.80-2.07 (m, 2H), 3.71 (p, *J* = 2.8 Hz, 1H), 4.06 (dd, *J* = 16.4 Hz, *J* = 21.4 Hz, 2H), 4.62 (td, *J* = 1.3 Hz, *J* = 5.7 Hz, 2H), 5.18-5.38 (m, 2H), 5.91 (tdd, *J* = 5.7 Hz, *J* = 10.3 Hz, *J* = 17.1 Hz, 1H).

¹³C-NMR (50 MHz, CDCl₃) : δ (ppm) 22.99, 23.46, 27.67, 31.15, 34.47, 39.08, 40.72, 48.97, 65.64, 66.07, 76.63, 118.94, 132.25, 171.11.

MS [e/m (%)] : 240 (M+, <1), 141 (81), 125 (18), 109 (35), 83 (91), 69 (86), 67 (26), 57 (23), 55 (55), 43 (23), 41 (100), 39 (26).

### trans-Isomer:

¹H-NMR (200 MHz, CDCl₃, selected data): δ (ppm) 1.46-1.63 (m, 1H), 3.48 (tt, *J* = 4.3 Hz, *J* = 11.4 Hz, 1H), 4.12 (s, 2H).

¹³C-NMR (50 MHz, CDCl₃, selected data): δ (ppm) 22.80, 26.13, 27.55, 33.54, 41.14, 44.92, 48.10, 65.73, 66.03, 76.98, 119.10.

MS [e/m (%)] : 240 (M+, <1), 141 (58), 125 (24), 109 (22), 83 (73), 69 (100), 67 (18), 57 (25), 55 (48), 43 (19), 41 (80), 39 (19).

### Example 4: (3Z)-Hex-3-enyloxy-acetic acid (3Z)-3-hexenyl ester

It is prepared from cis-3-hexenol and chloroacetic acid *cis*-3-hexenyl ester according to step 1 of example 1.
Odour description: clean (Ironed linen), green.

¹H-NMR (200 MHz, CDCl₃) : δ (ppm) 0.95 (t, *J* = 7.5 Hz, 6H), 1.95-2.14 (m, 4H), 2.26-2.46 (m, 4H), 3.52 (t, *J* = 7.1 Hz, 2H), 4.07 (s, 2H), 4.14 (t, *J* = 7.0 Hz, 2H), 5.20-5.60 (m, 4H).

¹³C-NMR (50 MHz, CDCl₃) : δ (ppm) 14.18, 14.26, 20.55, 20.59, 26.65, 27.62, 64.21, 68.24, 71.40, 123.33, 124.27, 133.94, 134.73, 170.47.

IR (film, cm-1): 723w, 995w, 1045w, 1070w, 1137s, 1195s, 1283m, 1430w, 1461w, 1738m, 1758s, 2876m, 2935m, 2965s, 3012m.

MS [e/m (%)]: 240 (M+, <1), 83 (44), 82 (90), 67 (74), 55 (100), 41 (46).

### Example 5: Fragrance composition containing the derivative obtained in example 1

A Freesia-type accord was prepared from the following ingredients:

| **Ingredients** | **Accord A** | **Accord B** |
|---|---|---|
| LINALOL | 260 | 260 |
| ETHYL LINALOL | 300 | 300 |
| LINALYL ACETATE | 50 | 50 |
| PHENYLETHYLIQUE ALCOOL | 100 | 100 |
| TERPINEOL LILLOL | 50 | 50 |
| METHYL DIHYDROJASMONATE | 30 | 30 |
| ALDEHYDE C14 10%DPG | 10 | 10 |
| | | |
| Hex-3-enyloxy-acetic acid allyl ester (Example 1) | 200 | 0 |
| | | |
| DPG | 0 | 200 |

Adding hex-3-enyloxy-acetic acid allyl ester to the accord (Accord A) generously impacts the freesia note, bringing a nice natural aspect and, moreover, power.
These 2 compositions, containing (A) or not (B) the hex-3-enyloxy-acetic acid allyl ester from example 1, were used as an alcoholic solution and to perfume a shower gel and a cream, at usual dilutions, known to the person of the art. Again, the impact of the addition of the hex-3-enyloxy-acetic acid allyl_ester in accord A is important, giving a nicer, more natural and very powerful fragrance. Accord A, containing hex-3-enyloxy-acetic acid allyl ester, even largely covers the odour of the cream base, on contrary to the accord B.

## Claims

1. A compound of formula (I) 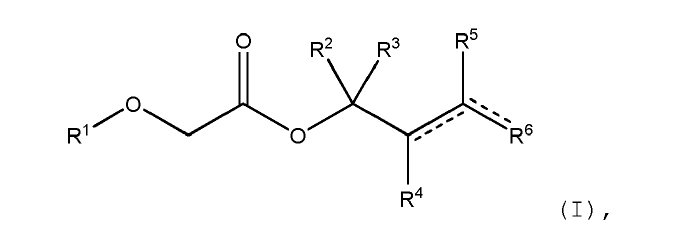 wherein
R¹ is C₆-C₁₂ alkenyl or a saturated or unsaturated C₆ carbocyclic group, wherein the carbocyclic group is substituted with at least one alkyl or alkenyl group,
R², R³, R⁴, R⁵, and R⁶ are independently H, C₁-C₅ alkyl, or C₂-C₅ alkenyl;
is a single or double bond, provided that one of the is a single bond and the other one is a double bond;
provided that if R⁶ is attached to the double bond, it is H, C₁-C₅ alkyl, or C₂-C₅ alkenyl; and
if R⁶ is part of the double bond, it is =CR'R", wherein R' and R" are independently H, C₁-C₄ alkyl, or C₂-C₄ alkenyl, with the proviso that R' and R" together form at most a C₄-alkyl or C₄-alkenyl group

2. The compound of claim 1, wherein R¹ is a cyclohexyl or cyclohexenyl group substituted with 1 to 4 groups individually selected from C₁-C₆ alkyl and C₂-C₆ alkenyl.

3. The compound of claim 1, wherein R¹ is selected from the group consisting of 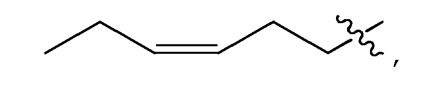 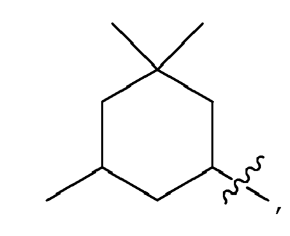 and

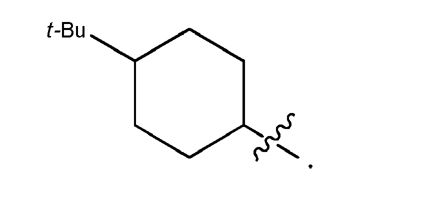

4. The compound of any of claims 1 to 3, wherein R², R³, R⁴, R⁵, and R⁶ are H or R², R³, R⁴, and R⁵ are H and R⁶ is =CR'R", wherein R' is H and R" is =CH-CH₂-CH₃.

5. Use of a compound according to any of claims 1 to 4, as a fragrant agent or as a flavouring agent.

6. Use of a compound according to any of claims 1 to 4, as a masking agent of odours and/or flavours.

7. Use according to claim 5 or 6 **characterized in that** the compound is comprised in a composition selected from pharmaceutical, cosmetic or food compositions.

8. Use according to any of claims 5 to 6 **characterized in that** the compound is used in combination with other perfuming or flavouring ingredients, solvents, additives or fixatives.
